# EUROPÄISCHE PATENTSCHRIFT

(11) **EP 3 113 699 B1**
(45) Veröffentlichungstag und Bekanntmachung des Hinweises auf die Patenterteilung: **16.08.2017**
(21) Anmeldenummer: 15706777.8
(22) Anmeldetag: 23.02.2015
(51) Int. Cl.: A61B 17/28, A61B 17/30, A61B 90/00

(54) **MIKROCHIRURGISCHES HALTE- UND/ODER SCHNEIDINSTRUMENT**
MICROSURGICAL HOLDING AND/OR CUTTING INSTRUMENT
INSTRUMENT MICRO-CHIRURGICAL DE COUPE ET/OU DE MAINTIEN

(30) Priorität: 27.02.2014 DE 102014102606
(43) Veröffentlichungstag der Anmeldung: 11.01.2017
(73) Patentinhaber: Aesculap AG, 78532 Tuttlingen (DE)
(72) Erfinder: STORZ, Stefan, 78532 Tuttlingen (DE); SCHWEITZER, Tom, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2015/053731
(87) Internationale Veröffentlichungsnummer: WO 2015/128291

(56) Entgegenhaltungen:
- DE-A1- 2 919 271
- DE-A1- 3 126 578
- DE-A1- 4 115 937
- DE-C- 144 979
- DE-C- 661 032
- US-A- 4 397 312
- US-A1- 2010 318 102
- US-A1- 2012 016 401
- US-A1- 2012 303 049

## Beschreibung

Die Erfindung betrifft ein (mikro-) chirurgisches Brancheninstrument vorzugsweise ein Handinstrument zum Greifen, Halten und/oder Schneiden beispielsweise von dünnen Gegenständen (Nadeln, Fäden, Drähte, etc.) oder von Körpergewebe. Insbesondere, aber nicht ausschließlich betrifft die Erfindung ein chirurgisches Handinstrument der Zangen- und/oder Scherenbauart nach dem Oberbegriff von Anspruch 1.

### Hintergrund der Erfindung

Für (mikro-) chirurgische Untersuchungen und Operationen werden Handinstrumente benötigt, die das Greifen, Halten und ggf. Schneiden von Gegenständen oder Körpergewebe im Patienten ermöglichen. Zur Betätigung der Handinstrumente sind hebeiförmige Handgriffe oder Griffschalen vorgesehen, die um einen gemeinsamen Schwenkbolzen aneinander scharniert und manuell gegeneinander schwenkbar sind für ein Aktuieren von daran gekoppelten Instrumentenbranchen. Entfällt die manuelle Betätigungskraft auf die Handgriffe/Griffschalen müssen diese wieder auseinandergedrückt werden, um die Instrumentenbranchen in deren Ausgangslage zurück zu bewegen. Dies erreicht man beispielsweise durch entsprechende instrumenteninterne Spannvorrichtungen, wie etwa biegeelastische Blattfedern. Diese Blattfedern können beispielsweise als externe Elemente zwischen den Handgriffen an diesen montiert sein oder als proximale Verlängerung des Instruments an den proximalen Enden der Handgriffe vorzugsweise einstückig mit diesen angebracht sein. Ungeachtet der jeweiligen Anordnung der Spannvorrichtung haben diese in der Regel gemeinsam, dass die freien Enden der an beiden Handgriffen jeweils angeordneten Blattfedern miteinander gekoppelt sind, wodurch eine Art Reihenschaltung von zwei Blattfedern ausgebildet wird.

Um das chirurgische Instrument u. a. in eine Reinigungsstellung bringen zu können, werden bei chirurgischen Handinstrumenten dieser Gattung, speziell bei Mikroscheren, Mikrozangen und Mikronadelhaltern, die freien Federenden in voneinander gelöstem Zustand aneinandergelegt, um so das chirurgische Handinstrument komplett über den Schwenk- oder Scharnierbolzen öffnen zu können. Zur lösbaren Kopplung / Konnektierung der freien Blattfederenden verwenden am Markt etablierte manuelle Mikroinstrumente sogenannte Durchsteckverbindungen. Bei einigen Durchsteckverbindungen dieser Art wird eine T-förmige Lasche am freien Ende der einen Blattfeder durch eine Rechtecköffnung am freien Ende der anderen Blattfeder durch Verdrehen der beiden Federenden durchgesteckt. Über die T-förmige Lasche sind die Federenden zwar formschlüssig gekoppelt, jedoch entsteht ein Formschluss mit viel Spiel, sodass die Federenden keine richtige Führung während der Betätigungsbewegung der beiden Handgriffe haben. Insoweit erfolgt die Führung der Blattfedern wie auch der Handgriffe ausschließlich am Scharnierbolzen, der demnach ohne großes Spiel in Schwenkbohrungen an den Griffschalen eingepasst sein sollte.

### Stand der Technik

Aus dem Stand der Technik ist beispielsweise eine mikrochirurgische Pinzette bekannt, wie sie in der DE 29 19 271 C2 beschrieben ist. In der DE 29 19 271 C2 wird folglich ein manuelles Pinzetteninstrument offenbart, das aus zwei über Band- bzw. Blattfedern mit proximalem (nicht lösbaren) Gelenk verbundenen Griffplatten oder Griffschalen besteht, die an Ihren jeweils distalen Ende eine Pinzetten-, eine Scheren- oder eine sonstige Greif- oder Haltebranche aufweisen. Die Federn sind dabei auswechselbar an den Griffschalen bzw. Griffplatten montiert. Das proximale Gelenk wird in diesem Fall durch einen Scharnierstift gebildet, der mit an den Bandfedern ausgebildeten Ösen verpresst ist. DE3126578, welches als nächstliegender Stand der Technik betrachtet werden kann, offenbart ein chirurgisches Handinstrument gemäß des Oberbegriffs von Anspruch 1.

Die bisher bekannten Lösungen haben jedoch die folgenden Nachteile:
- Die Reinigung und Aufbereitung der chirurgischen Handinstrumente ist bei nichtlösbarer Verbindung der beiden Blattfedern schwierig, da die Handgriffe nicht beliebig weit verschwenkt werden können, um eine möglichst geringe Überdeckung der Handgriffe und Instrumentenbranchen im Schwenkbolzenbereich zu erreichen.
- Durchsteckverbindungen nach dem bekannten Prinzip der T-förmigen Lasche und rechteck-förmiger Öffnung sind zwar vergleichsweise einfach zu entkoppeln, sie weisen jedoch in der Regel ein erhebliches Spiel auf. Sie sind daher zu Führung der freien Enden der Blattfedern und damit indirekt zur Führung der Handgriffe völlig ungeeignet.
- Ein chirurgischer Handschuh kann zudem bei Handhabung einer Durchsteckverbindung leicht eingeklemmt und damit beschädigt werden. Diese Gefahr besteht selbst bei einer chirurgischen Betätigung des Instruments.
- Durch starkes Verdrehen der Federenden beim Einführen der T-förmigen Lasche in die Rechteck-Öffnung kann es zu Materialbelastungen durch Torsion kommen, die ggf. eine plastische Verformung der Blattfedern bewirkt. Dadurch kann es sein, dass die beiden Handgriffe nicht mehr exakt parallel zueinander geschwenkt werden und dadurch verkanten.

### Kurzbeschreibung der Erfindung

Der Erfindung liegt angesichts der vorstehend erläuterten Problematik die Aufgabe zugrunde, eine einfach handhabbare und formschöne lösbare Verbindung für Blattfederenden an chirurgischen Handinstrumenten der vorstehend genannten Gattung zu schaffen. Ein bevorzugtes Ziel ist es, die lösbare Verbindung so zu gestalten, dass sie vergleichsweise einfach zu koppeln und zu entkoppeln ist. Ein weiteres bevorzugtes Ziel ist es, die lösbare Verbindung so zu gestalten, dass sie eine Führungsfunktion zumindest für die Blattfedern übernehmen kann.

Zur Lösung der gestellten Aufgabe wie auch ggf. zur Erreichung der bevorzugten Ziele wird ein chirurgisches Handinstrument der Zangen- oder Scherenbauart mit den Merkmalen gemäß dem Anspruch 1 vorgeschlagen. Bevorzugte Ausführungsformen und/oder Weiterbildungen der Erfindung, die ggf. auch unabhängig von den Merkmalen des Anspruchs 1 beansprucht werden können, sind Gegenstand der Unteransprüche.

Der Erfindung liegt der folgende Gedanke zugrunde:
Die aneinander anscharnierten hebelförmigen Griffe eines gebräuchlichen (mikro-) chirurgischen Handinstruments der Zangen- oder Scherenbauart (nicht Pinzettenbauart) sind durch zwei (proximal) daran angeordnete Blattfedern gegeneinander vorgespannt, deren jeweils freie Enden scharnierartig miteinander lösbar gekoppelt sind. Vorzugsweise sind die Blattfedern jeweils in Verlängerung eines zugehörigen der hebelförmigen Instrumentengriffe (Griffschale) in Richtung proximal sowie weiter vorzugsweise einstückig mit dem jeweils zugehörigen Instrumentengriff (Griffhebel) montiert oder ausgeformt. Die beiden Blattfedern (proximale Federabschnitte der beiden Handgriffe) weisen zumindest in Konstruktionslage und vorzugsweise bei nicht gekoppelten Federenden eine (vorgeformte) Bogenform (aufeinander zu) auf, um die beiden Griffe des chirurgischen Handinstruments nach Kopplung der freien Blattfederenden gegeneinander in Instrumentenöffnungsrichtung zu bringen / zu halten / vorzuspannen.

Durch die Lösbarkeit der scharnierförmigen Kopplung der proximalen freien Blattfederenden kann das Instrument grundsätzlich in eine Reinigungs- oder Sterilisationsstellung gebracht werden, indem die Kopplung/Verbindung gelöst wird und die Blattfedern/Federabschnitte damit außer Funktion gebracht werden.

Erfindungsgemäß besteht die Kopplung der beiden Blattfederenden prinzipiell aus wenigstens einem Scharnierstift an einem Blattfederende und wenigstens einer Aufnahmeöse am anderen Blattfederende. Die wenigstens eine Öse hat einen Innendurchmesser vorzugsweise mit einem gegenüber dem wenigstens einen Scharnierstift geringen Übermaß für das Erreichen einer im Wesentlichen spielfreien Drehführung des Scharnierstifts in der Öse. Dadurch kann die Blattfederkopplung die federelastischen Bewegungen der beiden Blattfedern und damit auch die Schwenkbewegung der beiden Griffe (indirekt) führen und so einem Verkanten der Griffe entgegenwirken.

Für ein Montieren der scharnierförmigen Blattfederkopplung hat diese wenigstens eine Öse einen durchgehenden Längsschlitz mit einer Schlitzweite, die ein Einführen des wenigstens einen Scharnierstifts erlaubt. Dabei ist der Längsschlitz vorzugsweise an einer Winkelposition ungleich der am weitesten proximalen Winkelposition der Öse platziert, wodurch verhindert wird, dass der Scharnierstift bei einer chirurgischen Betätigung des Instruments, bei welcher auch Kräfte in Richtung proximal auf den wenigstens einen Scharnierstift einwirken, aus der wenigstens einen Öse herausgedrückt wird.

Infolge der wenigstens einen längsgeschlitzten Öse ist das Zusammenführen der proximalen Federenden nach der Aufbereitung/Reinigung/Sterilisation und auch das erneute Entkoppeln vergleichsweise einfach, da der Scharnierstift nicht in Ösenlängsrichtung aus dieser herausgezogen bzw. eingesteckt werden muss, sondern durch entsprechendes Verformen wenigstens einer der Blattfedern (dadurch wird eine der Blattfedern gegenüber der anderen etwas verlängert oder verkürzt) gleitet der wenigstens eine Scharnierstift quasi selbständig aus dem/in den Längsschlitz. D.h. das Ein-/Auskuppeln erfolgt nicht mehr durch feinfühliges Manipulieren des wenigstens einen Scharnierstifts (mit Handschuhen praktisch unmöglich) sondern durch einfaches Drücken/Ziehen an zumindest einer der Blattfedern.

In anderen Worten ausgedrückt sind die beiden sich gegenüberliegenden Blattfedern zumindest in montiertem/scharniergekoppeltem Zustand ohne zusätzliche Betätigung (Konstruktionslage) bogenförmig und vorzugsweise in entkoppeltem Zustand bogenförmig vorgeformt. Die Position des Axialschlitzes ist in Abhängigkeit der (zumindest in Konstruktionslage sich ergebenden) Bogenform der beiden Blattfedern so gewählt, dass durch Drücken zumindest der einen Blattfeder mit Lagerstift vorzugsweise an einer vordefinierten/markierten Stelle in Richtung hin zur gegenüberliegenden anderen Blattfeder mit geschlitztem Lagerauge das Lagerauge durch dabei auftretendes Biegen der zugehörigen einen Blattfeder (mit Lagerauge) gedreht wird, bis der Lagerstift durch den Axialschlitz hindurch aus dem Lagerauge gleitet. Dies wird prinzipiell nur möglich, wenn sich erfindungsgemäß der Axialschlitz auf jener Seite (nachfolgend als Innenseite bezeichnet) der einen Blattfeder befindet, die der anderen Blattfeder (mit Lagerstift) zugewandt ist.

Anders ausgedrückt, ist die Position des Axialschlitzes so gewählt, dass die Blattfedern auskoppelbar sind, indem die Blattfeder mit Lagerstift durch Drücken, vorzugsweise an einer vordefinierten/markierten Stelle in Richtung hin zur gegenüberliegenden anderen Blattfeder mit geschlitztem Lagerauge, gekrümmt wird (d.h. der Radius der Bogenform der Blattfeder mit Lagerstift wird verkleinert), so dass durch die vom Lagerstift auf das Lagerauge übertragene Kraft auch die Blattfeder mit geschlitztem Lagerauge krümmt (d.h. auch der Radius der Bogenform der Blattfeder mit geschlitztem Lagerauge wird verkleinert), und die vom Lagerstift auf das Lagerauge übertragene Kraft beim Überschreiten eines Schwellenwerts ein Tordieren der Blattfeder mit geschlitztem Lagerauge und damit ein Verdrehen des geschlitzten Lagerauges bewirkt, so dass der Lagerstift durch den Axialschlitz hindurch aus dem Lagerauge gleitet. Dies wird prinzipiell nur möglich, wenn sich erfindungsgemäß der Axialschlitz auf der Innenseite der Blattfeder mit geschlitztem Lagerauge befindet.

Vorzugsweise ist die Position des Axialschlitzes gemäß der Erfindung im Wesentlichen distal gegenüberliegend zu der am weitesten proximalen Winkelposition des Lagerauges platziert.

Dadurch, dass der Lagerstift drehbar im Lagerauge gelagert ist, können die beiden Griffabschnitte mit den jeweiligen bogenförmigen Blattfedern derart aus der Konstruktionslage aufeinander zu bewegt werden, dass die Bogenform der Blattfedern jeweils erhalten bleibt und somit eine Rückstellkraft bewirkt wird, die mit dem Aufeinanderzubewegen der Griffabschnitte bzw. mit der Verkleinerung des Winkels zwischen den beiden Griffabschnitten linear, bzw. im Wesentlichen linear, zunimmt. Eine drehfeste Lagerung des Lagerstifts in dem Lagerauge würde dazu führen, dass die Blattfedern ausgehend von ihrer Bogenform in eine S-Form (Doppelbogenform mit Wendepunkt) gebracht werden, wenn die beiden Griffabschnitte aus der Konstruktionslage aufeinander zu bewegt werden. In diesem Fall nimmt die Rückstellkraft mit der Verkleinerung des Winkels zwischen den beiden Griffabschnitten nicht-linear zu.

Grundsätzlich lassen sich chirurgische Handinstrumente konstruktiv in Pinzetten- und Scherenmechanismen unterscheiden. Bei Pinzettenmechanismen verlaufen die Instrumentenhebel (jeweils bestehend aus Branche und Griff) im Wesentlichen parallel zueinander, wohingegen bei Scherenmechanismen sich die Instrumentenhebel kreuzen und an der Kreuzungsstelle aneinander scharniert sind. Das gattungsgemäße chirurgische Handinstrument zum Greifen, Klemmen, Zwicken oder Schneiden eines Gegenstandes oder eines Patientengewebes folgt dem vorstehend genannten Scherenmechanismus und hat demnach:
- zwei langgestreckte (sich kreuzende und aneinander scharnierte) Instrumenten-/Hebelelemente mit jeweils einem Griffabschnitt und einem Kopf- bzw. Branchenabschnitt,
- ein Hebelgelenk (Scharnier), das die beiden Hebelelemente zwischen ihrem Griffabschnitt und Branchenabschnitt miteinander drehbar/scharnierartig verbindet, wobei
- sich an jeden der beiden Griffabschnitte jeweils ein Federabschnitt (Blattfeder) an deren proximalen Enden anschließt zum mechanischen Verbinden oder Koppeln der beiden Griffabschnitte, so dass die beiden Hebelelemente in einer offenen Instrumentenstellung (in Konstruktions- bzw. Ausgangslage) gehalten werden.

Die hierfür vorgesehene Scharnierkonstruktion an den proximalen Blattfederenden hat wenigstens zwei Scharnierelemente, nämlich wenigstens einen mit dem einen Federabschnitt an dessen proximalem Ende fest (vorzugsweise einstückig) verbundenen Scharnierstift und eine/ein mit dem anderen Federabschnitt an dessen proximalem Ende fest (vorzugsweise einstückig) verbundene Öse oder Lagerauge, wobei
- die Öse oder das Lagerauge in einer offenen/geschlitzten Bauweise ausgeführt ist, derart, dass die beiden Scharnierelemente ohne weitere Montagemaßnahmen, d. h. ohne Umbauten über den Längsschlitz in und außer Schwenkkopplung bringbar sind und wobei
- der Längs- oder Axialschlitz bevorzugt auf der Innenseite der zugehörigen Blattfeder (gemäß der vorstehenden Definition) angeordnet ist.

Die erfindungsgemäße Weiterentwicklung des gattungsgemäßen chirurgischen Handinstruments besteht in der Ausbildung des Scharnierstifts als ein an seinen beiden Enden mit dem einen Federabschnitt fest verbundenen Haltestift an einem freien, gegabelten Ende des einen Federabschnitts (geschlossene Bauform) und einer Öse/Rolle an einem freien proximalen Ende des anderen Federabschnitts, wobei die Rolle den (Längs-) Schlitz aufweist (offene Bauform) zum radialen Einführen des Scharnierstifts in die Rolle, so dass die Rolle den Haltestift (teilweise/mindestens dreiviertel-kreisförmig) umschließt.

Bevorzugte Ausführungsformen des chirurgischen Handinstruments weisen als einzeln sowie unabhängig beanspruchbares Merkmal oder Merkmalkombination auf, dass
- der Schlitz in der Rolle bezüglich der Rollenlängsachse schräg verläuft, so dass beim Einführen des Scharnierstifts in die Rolle wenigstens ein Federabschnitt (zusätzlich) elastisch verdreht werden muss;
- der Schlitz in der Rolle im Wesentlichen parallel zur Rollenlängsachse verläuft und sich in Richtung hin zu den Branchenabschnitten öffnet;
- die Federabschnitte für ein Koppeln vorgespannt werden müssen, so dass der Haltestift in der Rolle gehalten wird und gegen die Vorspannkraft (in Instrumentenlängsrichtung hin zu den Branchenabschnitten) verschoben werden müssen (unter Biegen zumindest eines Federabschnitts), um die Schwenkkopplung (Verbindung) der Federabschnitte zu lösen;
- der wenigstens eine Scharnierstift im Querschnitt unrund mit wenigstens einer Abflachung ausgebildet ist, wohingegen die Schlitzweite so eng gewählt ist, dass der Scharnierstift nur längs (im Bereich) seiner wenigstens einen Abflachung durch den Schlitz führbar ist.
- die Rolle federelastisch aufspreizbar ist, um die Schlitzweite für ein Durchführen des wenigstens einen Scharnierstifts aufzuweiten,
- zumindest eine der beiden Blattfedern (Federabschnitte) einen markierten Betätigungspunkt in einem Mittenabschnitt der Blattfeder aufweist, in dessen Bereich eine manuelle Druckkraft auf die Blattfeder aufbringbar ist, um diese in vordefinierter Weise zu biegen, derart, dass durch die entsprechend der Platzierung des Betätigungspunkts bewirkte Biegung der einen Blattfeder eine Relativdrehung des wenigstens einen Scharnierstifts bezüglich der Öse erfolgt, bis dieser infolge der dadurch einhergehenden Verkürzung der einen Blattfeder gegenüber der anderen quasi selbsttätig durch den Längsschlitz aus der Öse gleitet
- die Griffabschnitte jeweils einen teilkreisförmigen Querschnitt kleiner als ein Halbkreis aufweisen, jeweils an den einander zugewandten Seiten Aushöhlungen haben, in denen zusammenwirkende Bauteile einer Umlaufsperre zumindest teilweise aufgenommen sind und zwischen sich bei verrasteter Umlaufsperre einen Spalt oder Abstand definieren, dessen Spaltweite in Zusammenwirken mit den beiden Teilkreisen einen Vollkreis mit im wesentlichen konstantem Radius bilden.

Das erfindungsgemäße chirurgische Handinstrument hat die folgenden Vorteile:
- Es erfüllt die Anforderung der Anwender, die nach einer einfach handhabbaren und formschönen, lösbaren Verbindung für Federenden an chirurgischen Instrumenten verlangen.
- Bei dem erfindungsgemäßen Scharniergelenk am proximalen Ende der Federabschnitte weist die Schwenkkopplung/Verbindung ein geringes/nahezu kein Spiel auf, da das Einsetzen des Scharnierstifts in das Lagerauge radial über den Längsschlitz am Lagerauge erfolgt. Daher ist die Gefahr, dass chirurgische Handschuhe bei der Handhabung eingeklemmt werden, gering.
- Die Handhabung des chirurgischen Handinstruments ist einfach, da keine separaten Scharnierelemente wie Schwenkstifte eingesetzt werden müssen, sondern alle Scharnierelemente fest mit den Federabschnitten verbunden sind.
- Die Lösung präsentiert sich optisch hochwertig.
- Der Aufwand, mit der die wenigstens eine Feder manipuliert werden muss, um die Federenden zu verbinden, ist gering, so dass auch die Gefahr klein ist, die Federenden zu verbiegen bzw. zu beschädigen.

### Figurenbeschreibung

Weitere Merkmale und Vorteile der Erfindung ergeben sich aus der folgenden Beschreibung, bei der Bezug genommen wird auf die beigefügten Figuren.
Fig. 1 zeigt ein erstes Ausführungsbeispiel des erfindungsgemäßen (mikro-) chirurgischen Handinstruments in perspektivischer Darstellung in geöffnetem Zustand mit noch nicht vorgebogenen Blattfederabschnitten (Halbzeug),
Fig. 2 zeigt das erste Ausführungsbeispiel aus einer anderen Perspektive,
Fig. 3 zeigt das erfindungsgemäße proximale Scharnier der Blattfederabschnitte/Federelemente gemäß der Fig. 1 in vergrößerter Darstellung sowie in gekoppeltem/eingehaktem Zustand,
Fig. 4 zeigt das erfindungsgemäße Handinstrument gemäß der Fig. 1 in Seitenansicht mit eingekoppeltem proximalem Scharnier sowie in geschlossenem/betätigten Zustand,
Fig. 5 zeigt das erfindungsgemäße proximale Scharnier der Federelemente gemäß der Fig. 1 in vergrößerter Darstellung sowie in ausgehaktem Zustand (Blattfedern sind auch hier noch nicht plastisch vorgebogen),
Fig. 6 zeigt eine weitere Ausführungsform des erfindungsgemäßen proximalen Scharniers mit zur Ösenachse schräggestelltem Längsschlitz sowie mit den beiden vollständigen Hebelelementen in aufgeklapptem Zustand (gelöstes distales Scharnier) in perspektivischer Darstellung,
Fig. 7 zeigt die Ausführungsform nach Fig. 6 in geschlossenem/betätigtem Zustand,
Fig. 8 zeigt in Perspektivenansicht das erfindungsgemäße chirurgische Instrument gemäß der Fig. 1, d.h. gemäß dem ersten bevorzugten Ausführungsbeispiel der vorliegenden Erfindung (jedoch im Gegensatz zu den Fig. 1 bis 9) in fertigem Zustand, in welchem die beiden Blattfederelemente bogenförmig zueinander (plastisch) vorgeformt sind, wobei jedoch das proximale Scharnier ausgehakt ist, und

Fig. 9 zeigt in Seitenansicht das erfindungsgemäße chirurgische Instrument in fertigem Zustand gemäß der Fig. 8, in welchem das proximale Scharnier ausgehakt ist.

Die Figuren sind nicht maßstäblich. Gleiche oder gleich wirkende Elemente sind für alle Ausführungsbeispiele mit denselben Bezugszeichen versehen, soweit nichts anderes erwähnt ist.

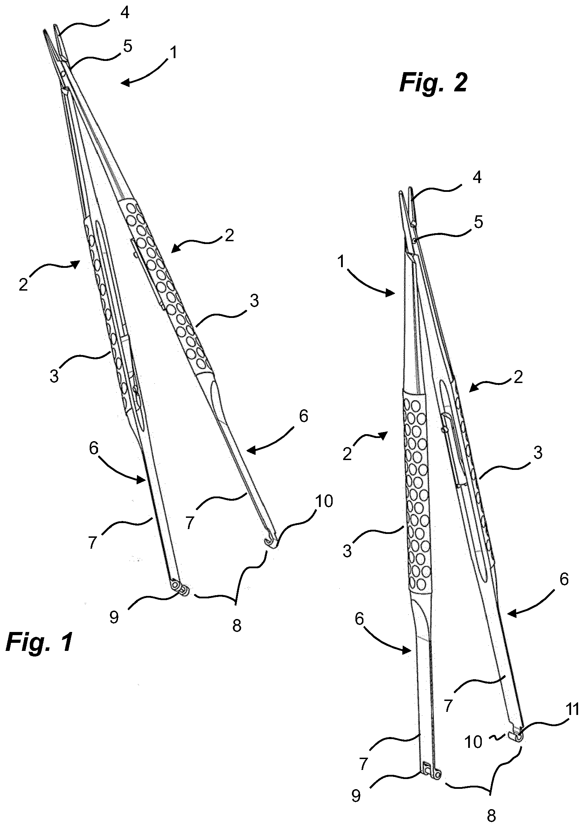

Der erfindungsgemäße Kopplungsmechanismus (auch als Verschlussmechanismus zu bezeichnen) zum federelastischen Koppeln der Betätigungshebel / Instrumentengriffe eines (mikro-) chirurgischen Handinstruments vorzugsweise der Zangen- oder Scherenbauart, so dass die von seinem Branchenabschnitt gebildete Maulöffnung federelastisch geöffnet ist, wird im Folgenden anhand der Fig. 1 bis 9 erläutert.

In Fig. 1 ist ein chirurgischen Handinstrument 1 der Zangen-/Scherenbauart gezeigt, das zwei Hebelelemente 2 aufweist, welche über ein Gelenk / distales Scharnier 5 dreh-/schwenkbar miteinander verbunden sind. Die Hebelelemente 2 weisen jeweils einen proximalen Griffabschnitt (Instrumentengriffe) 3 und einen distalen Branchen- oder Maulabschnitt 4 (Maulteil) auf. Zwischen dem Griffabschnitt 3 und dem Maulabschnitt 4 befindet sich das Gelenk / distale Scharnier 5, so dass sich bei Betätigen (aufeinander zu Bewegen) der (integralen) Griffabschnitte 3 der Hebelelemente 2 um das distale Scharnier 5 eine zangen- oder scherenartige Schließbewegung der distalen Maulabschnitte 4 ergibt. Dieser Zangen-Scherenmechanismus ist aus der Stand der Technik hinlänglich bekannt, sodass auf eine detaillierte Funktionsbeschreibung an dieser Stelle verzichtet wird.

Um die Möglichkeit einer Beibehaltung der Hebelelemente 2 in einer (geöffneten bzw. auseinander gehaltenen) Position zu schaffen, in welcher von dem Maulabschnitt 4 keine Klemmkraft ausgeübt wird, werden die beiden Griffabschnitte 3 an deren freien Enden jeweils durch eine (Blatt-)Feder 6 in Richtung proximal verlängert. Jede Feder 6 ist vorzugsweise ein Metallblatt, insbesondere aus Federstahl, das sich verhältnismäßig leicht und in einem ausreichenden Maße federelastisch biegen und - in Richtung des Hebelelements 2 gesehen - verdrillen lässt. Jedes Metallblatt 6 erstreckt sich dabei im Wesentlichen co-axial zum jeweiligen hebelförmigen Griffabschnitt 3 und ist fest mit diesem verbunden bzw. verbindbar. Beispielsweise ist jedes Metallblatt 6 mit dem zugehörigen Griffabschnitt stoßverlötet, verschweißt, verschraubt oder sogar stoffeinstückig ausgebildet.

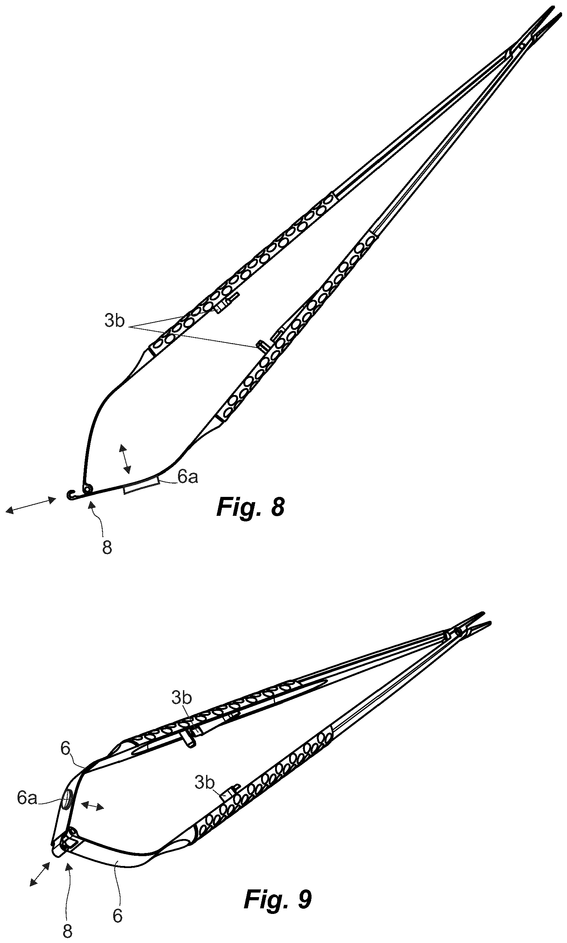

An dieser Stelle sei darauf hingewiesen, dass die beiden Blattfederabschnitte 6 gemäß der Fig. 1 wie auch gemäß der weiteren Figuren 2 bis 7 zunächst gerade dargestellt sind, sich also noch im halbfertigen Zustand befinden. In den Fig. 8 und 9 sind die beiden Blattfederabschnitte 6 hingegen bogenförmig dargestellt, also in Bogenform plastisch vorgeformt. Dies stellt den fertigen Zustand der Blattfedern 6 dar, in welchem die Blattfedern 6 eine gegen die manuelle Betätigung gerichtete Vorspankraft auf die Griffabschnitte 3 ausüben, welche das Instrument in Offenposition drückt/hält.

Das chirurgische Handinstrument 1 nach Fig. 1 ist in einer anderen Perspektive in Fig. 2 gezeigt.

Aus den Fig. 1 und 2 lässt sich der Materialdickenunterschied zwischen dem Griffabschnitt 3 und dem Federabschnitt 6 und damit ein Eindruck der elastischen Flexibilität des Federabschnitts 6 gegenüber dem (starren) Griffabschnitt 3 erkennen. Demnach sind die Federabschnitte 6 als Blattfedern ausgebildet und damit in eine Richtung (auf einer Ebene) elastisch biegbar, wohingegen die Federabschnitte in einer senkrecht hierzu stehenden Ebene starr sind. Damit können die Federelemente 6 per se grundsätzlich eine Führungsfunktion für eine Schwenkbewegung um eine Achse, senkrecht zur Biegeebene übernehmen.

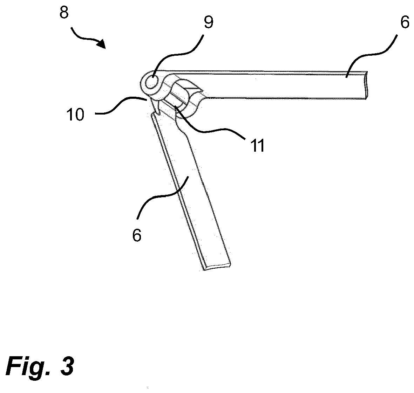

Die Federabschnitte 6 sind an einem distalen Ende mit dem jeweiligen Griffabschnitt 3 verbunden, wie dies vorstehend bereits beschrieben wurde, ihr anderes proximales Ende ist zunächst frei. An diesem freien proximalen Ende 7 der Federabschnitte 6 ist erfindungsgemäß ein Scharniergelenk 8 vorgesehen, das aus zwei Komponenten besteht, die zum Verbinden der beiden Hebelelemente 2 bzw. der Federabschnitte 6 zusammenwirken. Die Komponenten des Scharniergelenks 8 werden im Folgenden anhand von Fig. 3 beschrieben.

In Fig. 3 sind die zwei Federabschnitte 6 endseitig gezeigt, an deren proximale freie Enden jeweils eine Komponente des erfindungsgemäßen Scharniergelenks 8 angeordnet ist. Der gemäß Fig. 3 obere eine Federabschnitt 6 endet in einer Halterung mit einem Scharnierstift 9. Im Konkreten bildet der eine Federabschnitt 6 an seinem proximalen, gegabelten Ende zwei sich gegenüberliegende Lagerböcke, zwischen denen ein Scharnierstift 9 quer zur Längserstreckung des einen Federabschnitts 6 sowie quer zur Biegeebene des Federabschnitts 6 fest eingesetzt ist.

Der andere (gemäß Fig. 3 untere) Federabschnitt 6 endet dementsprechend in einem Lagerauge (Öse) 10, das einen im vorliegenden Beispiel parallel zur Lageraugenachse verlaufenden Längsschlitz 11 aufweist. Im Konkreten ist der andere Federabschnitt 6 an seinem proximalen Ende zu einer Rolle 10 umgebogen, die jedoch nicht geschlossen ist sondern einen Spalt oder Schlitz 11 definiert, der sich radial in Richtung hin zum Branchenabschnitt öffnet, d.h. allgemein an einer Winkelposition liegt, die ungleich ist zu der am weitesten proximal liegenden Winkelposition der Rolle 10.

Über den (Längs-)Schlitz 11 wird der Scharnierstift 9 unter Biegen des einen Federabschnitts 6 in die Rolle 10 radial eingeführt, so dass die Rolle 10 den Scharnierstift 9 (zumindest dreiviertelkreisförmig) umschließt und außer einer Drehbewegung eine Bewegung zwischen den beiden Federabschnitten 6 in Radial- oder Axialrichtung der Rolle 10 unmöglich gemacht wird. In gleicher Weise kann der Scharnierstift 9 wieder aus er Rolle 10 herausgenommen werden, ohne dass separate Bauteile aus dem Scharniergelenk gezogen und aufbewahrt werden müssen. Die Handhabung des Handinstruments ist daher sehr einfach.

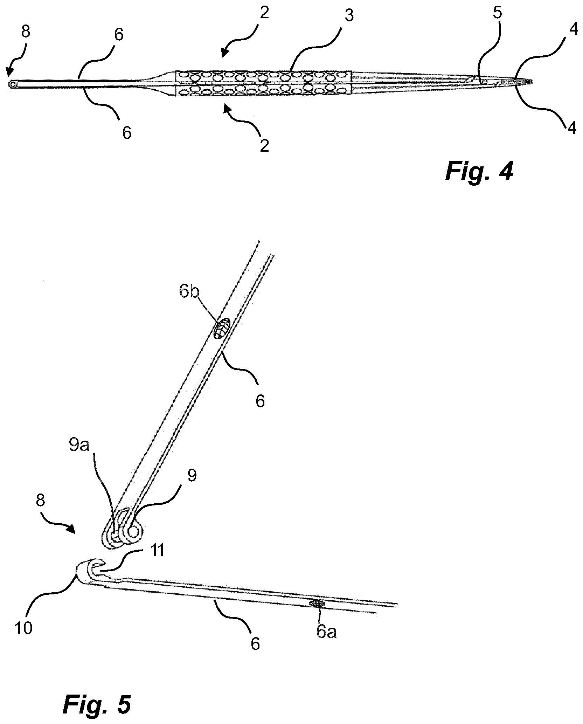

Fig. 4 zeigt das erfindungsgemäße Zangeninstrument in Seitenansicht in geschlossenem/betätigtem Zustand, in welchem das distale (Scheren-/Zangen-) Scharnier montiert ist. Aus diesem geschlossenen Zustand wird das Zangeninstrument dann durch die Federkraft der beiden gekoppelten Federabschnitte 6 mit Freigeben der Griffabschnitte 3 wieder geöffnet.

Zur genaueren Erläuterung ist das Scharniergelenk 8 in Fig. 5 in ausgehaktem Zustand gezeigt. Wie erkennbar ist, wird der Federabschnitt 6 mit der Rolle 10 in Richtung ihres Schlitzes 11 bewegt bzw. der Federabschnitt 6 mit dem Scharnierstift 9 radial in den Schlitz 11 hinein geschoben. Der Schlitz 11 ist in diesem Ausführungsbeispiel etwas enger als der Durchmesser des Scharnierstifts 9, so dass er mit einem gewissen Kraftaufwand über den Scharnierstift 9 an dem proximalen Ende des anderen Federabschnitts 6 gedrückt werden muss. Diese Engstelle hat den Vorteil, dass ein unbeabsichtigtes Lösen des Scharniergelenks 8 im praktischen Betrieb ausgeschlossen ist. Hierzu gibt es aber eine beliebige Anzahl von Alternativen.

Eine bevorzugte Alternative sieht vor, den Scharnierstift 9 mit einer Abflachung 9a an seinem Umfang auszubilden (dies ist in der Fig. 5 angedeutet) oder den Stiftquerschnitt unrund, z.B. entsprechend einer Ellipse auszubilden. In diesem Fall ist die Schlitzweite an so gewählt, dass der Stift nur im Bereich seiner Abflachung 9a in die Rolle 10 einführbar ist. Die Winkelposition der Abflachung 9a ist wiederum so gewählt, dass diese über den normalen Schwenkwinkelbereich des Instruments hinweg nicht mit dem Schlitz 11 übereinstimmt. Auf diese Weise kann ein Herausrutschen des Stifts 9 während eines chirurgischen Instrumentengebrauchs vermieden werden.

Wie aus der Fig. 5 ferner zu entnehmen ist, sind an den voneinander abgewandten Flachseiten der Blattfederabschnitte 6 Markierungen (Drucktasten) 6a, 6b in jeweils einem Längsmittenabschnitt der Blattfedern 6 vorgesehen. Diese Markierungen 6a, 6b können ggf. durch farbliche Aufdrucke oder Oberflächenveränderungen wie Aufrauungen, Rippen, etc. erzeugt sein und definieren jeweils Druckpunkte. Dabei sei darauf hingewiesen, dass die entsprechende Markierung nur einer der beiden Blattfedern 6 vorzugsweise jener mit der Rolle 10 für die nachfolgend beschriebene Funktion ausreichend ist.

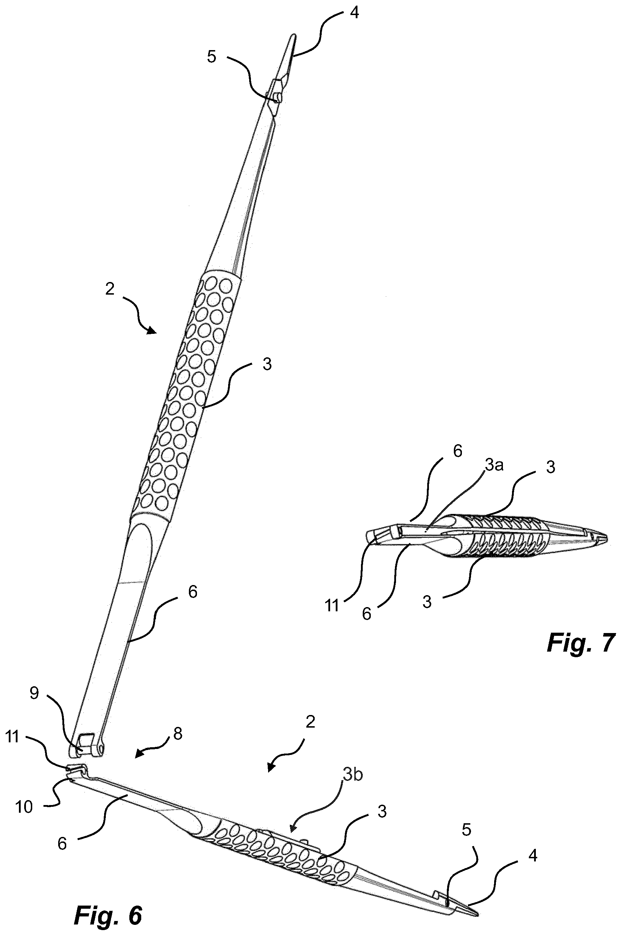

Die Erfindung ist nicht auf die Darstellung in Fig. 1 bis 5 beschränkt. Insbesondere sind verschiedene Formen in Bezug auf die Ausführung der Rolle 10 mit Schlitz 11 denkbar. Eine derartige Alternative zeigen Fig. 6 und 7.

Die Hauptkomponenten des erfindungsgemäßen chirurgischen Handinstruments in Fig. 6 und 7 sind bereits in den vorangehenden Figuren erläutert worden. Bei der Ausführungsform des Handinstruments nach Fig. 6 ist die Rolle 10 anders als bei den vorherigen Ausführungsformen nicht in Richtung des Branchenabschnittes 4 durch den axialen Schlitz 11 geöffnet, sondern stattdessen in entgegengesetzter Richtung schlitzförmig geöffnet. Ferner ist der Schlitz 11 gegenüber dem Federabschnitt 6 bzw. bezüglich der Rollenlängsachse (leicht) geneigt bzw. schräg verlaufend. Das bedeutet, dass der Federabschnitt 6 mit der Rolle 10 und/oder der Federabschnitt 6 mit dem Scharnierstift 8 leicht (um die Längsachse der Hebelelemente 2) verdrillt werden muss, um den Scharnierstift 9 in den Schlitz 11 einzuführen und damit das Scharniergelenk 8 zusammenzusetzen. Wenn der Federabschnitt 6 anschließend losgelassen wird, so dass er sich wieder entdrillt, rutscht der Scharnierstift 9 durch den Schlitz 11 in die Rolle 10. Dabei sei darauf hingewiesen, dass der Scharnierstift 9 in diesem Fall keine Abflachung braucht und die Schlitzweite dem Stiftdurchmesser entsprechen kann.

Das Handinstrument gemäß der Fig. 6 in geschlossenem Zustand ist in Fig. 7 gezeigt. Dabei zeigt sich ein weiteres Merkmal des erfindungsgemäßen Handinstruments, das im Übrigen für alle Ausführungsbeispiele gilt:
Wie dargestellt sind die Griffabschnitte 3 vorliegend schalenförmig ausgebildet, wobei jeder Griffabschnitt 3 im Querschnitt einen Teilkreis kleiner einem Halbkreis ausformt. Die Schalenform dient dazu, die Bauteile einer an sich bekannten Umlaufsperre 3b aufzunehmen, nämlich insbesondere ein Nockenbauteil an dem einen Griffabschnitt und ein damit zusammenwirkendes Kulissenbauteil an dem anderen Griffabschnitt. Umlaufsperren dieser Art zählen zum allgemein bekannten Stand der Technik und brauchen daher an dieser Stelle nicht näher beschrieben zu werden.

Erfindungsgemäß ist es nunmehr vorgesehen, den Teilkreis jedes Griffabschnitts 3 auf die verwendete Umlaufsperre bekannten Aufbaus abzustimmen, derart, dass im geschlossenen, sowie verrasteten Zustand des Instruments gemäß der Fig. 9 ein Vollkreis im Bereich der Griffabschnitte 3 entsteht, gebildet durch die Teilkreise der beiden Griffabschnitte 3 sowie einem Zwischenspalt 3a zwischen dem beiden Griffschalen 3, der funktionsbedingt zur Betätigung der aktuell verwendeten Umlaufsperre 3b erforderlich ist. Eine solche Vollkreisform mit im Wesentlichen konstantem Radius hat sich bei der Handhabung des Instruments als vorteilhaft gezeigt, da das geschlossene und verrastete Instrument beliebig um dessen Längsachse sicher und gleichmäßig gedreht werden kann, ohne dass das Instrument zwischen den Fingern kippt. Damit wird auch sichergestellt, dass das Instrumentenmaulteil 4 sicher geführt werden kann.

In den Fig. 8 und 9 ist das erfindungsgemäße Instrument in fertigem sowie montiertem Zustand gezeigt, wobei auch die beiden Bauteile der Umlaufsperre 3b angedeutet sind.

Demnach sind die beiden Federabschnitte 6 zu einer Bogenform plastisch verformt, um die Griffabschnitte 3 auseinander zu drücken. Werden in diesem Zustand, in welchem das proximale Scharnier 8 eingekoppelt ist, die beiden Griffabschnitte 3 gegeneinander gedrückt, erfahren die zwei Blattfederelemente 6 eine elastische Verbiegung/Streckung und verschwenken dabei im proximalen Scharnier 8 gegeneinander. Bei Freigeben der Griffabschnitte 3 drücken die beiden Blattfederelemente 6 das Instrument in seine Offenstellung zurück. Erfolgt die manuelle Betätigung über einen bestimmten Schwenkpunkt hinweg, rastet die Umlaufsperre 3b ein und hält so das Instrument in seiner Schließposition.

Um das proximale Scharnier 8 mit nicht geneigtem Schlitz 11 gemäß dem ersten bevorzugten Ausführungsbeispiel der vorliegenden Erfindung zu entkoppeln, wird wenigstens das eine Blattfederelement mit endseitiger Rolle 10 im Bereich der Markierung 6a seitwärts eingedrückt. Dadurch verbiegt sich dieses Federelement wodurch die Rolle 10 bezüglich des Scharnierstifts 9 zu drehen beginnt. Gleichzeitig streckt sich dieses eine Blattfederelement und übt so eine Druckkraft auf die Rolle 10 in Richtung proximal aus.

Bei einer bestimmten Betätigungsstrecke kommt der Längsschlitz 11 im Bereich der Abflachung 9a des Scharnierstifts 9 zu liegen und die Rolle 10 wird in Richtung proximal über den Scharnierstift 9 verschoben. Damit ist das proximale Scharnier 8 entkoppelt.

Zusammenfassend zeichnet sich die Erfindung in den folgenden Aspekten gegenüber dem Stand der Technik aus:
Bei der vorliegenden Erfindung wird die Verbindung/Schwenkkopplung der beiden Federabschnitte 6 durch ein solches Scharniergelenk 8 realisiert, das ohne vorstehende Laschen für die Verbindung der Federenden auskommt.

In einem Ausführungsbeispiel ist das Lagerauge/die Rolle 10 des Scharniers 8 schräg geschlitzt, so dass durch leichtes Verdrehen/Verdrillen zumindest des einen Federendes der Stift 9 des Scharniers 8 über den Schlitz 11 aus-/eingeführt werden kann. In einem weiteren Ausführungsbeispiel ist das Lagerauge/die Rolle 10 des Scharniers so geschlitzt, dass der Stift 9 in Richtung hin zum Branchenabschnitt entlang der Blattfeder zum Lösen geführt werden muss, wobei der eine Federabschnitt 6 elastisch gebogen wird. Über die Federspannung beider Federabschnitte 6 und dem Formschluss (Scharnierstift 9 in der Rolle 10) wird der Stift 9 in seiner drehbaren Funktionsstellung in der Rolle 10 des Scharniers 8 gehalten. Als weiteres Ausführungsbeispiel ist die Schwenkkopplung/Verbindung als Schnapp-/Klickverbindung ausführbar.

Die Erfindung betrifft zusammenfassend ein chirurgisches Handinstrument 1 zum Greifen eines Gegenstandes, das umfasst: zwei Hebelelemente 2 mit jeweils einem Griffabschnitt 3 und einem Maul-/Branchenabschnitt 4, einem Hebelgelenk 5, das die beiden Hebelelemente zwischen ihrem Griffabschnitt und Branchenabschnitt miteinander drehbar verbindet, wobei sich an jeden der beiden Griffabschnitte jeweils ein Federabschnitt 6 anschließt zum Verbinden der beiden Griffabschnitte, so dass die beiden Hebelelemente 2 in einer offenen Stellung federelastisch gehalten werden. Zur Schaffung einer einfach handhabbaren und formschönen lösbaren Verbindung für Federenden an chirurgischen Instrumenten umfasst das Handinstrument einen fest damit verbundenen Scharnierstift 9 an einem freien Ende 7 des einen Federabschnitts und ein fest damit verbundenes Lagerauge 10 an einem freien Ende 7 des anderen Federabschnitts, wobei das Lagerauge einen Längsschlitz 11 aufweist zum radialen Einführen des Scharnierstifts 9 in das Lagerauge 10.

### Bezugszeichen

- 1: Handinstrument
- 2: Hebelelement
- 3: Griffabschnitt
- 3a: Spalt
- 3b: Umlaufsperre
- 4: Maulabschnitt
- 5: Hebelgelenk/distales Scharnier
- 6: Federabschnitt
- 6a, b: Betätigungsmarkierung/Taste
- 7: Proximales, freies Ende von Federabschnitt
- 8: Scharniergelenk am proximalen Federende
- 9: Scharnierstift
- 10: Rolle
- 11: Schlitz

## Patentansprüche

1. Chirurgisches Handinstrument (1) der Zangen- oder Scherenbauart, das folgende Teile hat:
zwei aneinander scharnierte Hebelelemente (2) mit jeweils einem Griffabschnitt (3) auf einer Seite eines Hebelelement-Scharniers (5) und einem Maulabschnitt (4) auf der anderen Seite des Hebelelementscharniers (5),
zwei Blattfedern (6), die sich an jeden der beiden Griffabschnitte (3) als diese jeweils verlängernde Federabschnitte proximal anschließen und an ihren proximalen freien Enden über ein Scharniergelenk (8) miteinander gekoppelt sind,
**dadurch gekennzeichnet, dass**
das Scharniergelenk (8) einen Scharnierstift (9) am freien Ende der einen Blattfeder (6) und wenigstens ein Lagerauge (10) am freien Ende der anderen Blattfeder (6) aufweist,
wobei das Lagerauge (10) einen in Lageraugen-Achsrichtung verlaufenden Schlitz (11) aufweist zum radialen Einführen des Scharnierstifts (9) in das Lagerauge (10).

2. Chirurgisches Handinstrument (1) nach Anspruch 1,
**dadurch gekennzeichnet, dass**
der Längsschlitz (11) in einer Winkelposition ungleich der am weitesten proximalen Winkelposition des Lagerauges (10) vorzugsweise in einem distalen Winkelpositionsbereich und weiter vorzugsweise im Wesentlichen gegenüberliegend zur am weitesten proximalen Winkelposition des Lagerauges (10) platziert ist.

3. Chirurgisches Handinstrument (1) nach Anspruch 1 oder 2,
**dadurch gekennzeichnet, dass**
der Längsschlitz (11) in dem Lagerauge (10) bezüglich der Lageraugenachse schräg verläuft, so dass beim radialen Einführen des Scharnierstifts (9) in das Lagerauge (10) wenigstens ein Federabschnitt (6) verdrillt werden muss.

4. Chirurgisches Handinstrument (1) nach einem der vorstehenden Ansprüche,
**dadurch gekennzeichnet, dass**
die Schlitzweite kleiner ist als der maximale Durchmesser des Scharnierstifts (9).

5. Chirurgisches Handinstrument (1) nach Anspruch 4,
**dadurch gekennzeichnet, dass**
das Lagerauge (10) federelastisch aufweitbar ist.

6. Chirurgisches Handinstrument (1) nach Anspruch 4 oder 5,
**dadurch gekennzeichnet, dass**
der Scharnierstift (9) wenigstens eine Abflachung (9a) aufweist, wobei der Scharnierstift-Durchmesser im Bereich der wenigstens einen Abflachung im Wesentlichen der Schlitzweite entspricht oder kleiner ist.

7. Chirurgisches Handinstrument (1) nach einem der vorstehenden Ansprüche,
**dadurch gekennzeichnet, dass**
wenigstens einer der Federabschnitte (6), vorzugsweise der Federabschnitt (6) mit dem endseitigen Lagerauge (10) in seinem Längsmittenabschnitt eine Markierung (6a) an dessen dem anderen Federabschnitt 6) abgewandten Seite trägt, in deren Bereich eine äußere Druckkraft für ein unmittelbar daraus resultierendes Auskoppeln des proximalen Scharniers (8) manuell anzulegen ist.

8. Chirurgisches Handinstrument (1) nach Anspruch 5 und 6,
**dadurch gekennzeichnet, dass**
die Winkelposition der Abflachung (9a) bezüglich der Winkelposition des Schlitzes (11) so gewählt ist, dass
- bei einer chirurgischen Instrumentenbetätigung keine Übereinstimmung beider Winkelpositionen möglich ist und
- bei einer Druckkraftbeaufschlagung wenigstens des einen Federabschnitts (6) an der Markierung (6a) eine Übereinstimmung beider Winkelpositionen für ein Entkoppeln des proximalen Scharniers (8) erreicht wird.

9. Chirurgisches Handinstrument (1) nach einem der vorstehenden Ansprüche,
**dadurch gekennzeichnet, dass**
die Griffabschnitte (6)
- jeweils einen teilkreisförmigen Querschnitt kleiner als ein Halbkreis aufweisen,
- jeweils an den einander zugewandten Seiten Aushöhlungen haben, in denen zusammenwirkende Bauteile einer Umlaufsperre (3b) zumindest teilweise aufgenommen sind und
- zwischen sich bei verrasteter Umlaufsperre (3b) einen Spalt oder Abstand (3a) definieren, dessen Spaltweite in Zusammenwirken mit den beiden Teilkreisen einen Vollkreis mit im Wesentlichen konstantem Radius bilden.

10. Chirurgisches Handinstrument (1) nach einem der vorstehenden Ansprüche
**dadurch gekennzeichnet, dass**
die Federabschnitte (6) in montiertem Zustand des Scharniergelenks (8) gegeneinander vorgespannt sind, so dass der Scharnierstift (9) in dem Lagerauge (10) durch die Vorspannkraft gehalten wird und gegen die Vorspannkraft aus dem Schlitz (11) verschoben werden muss, um die Scharnierkopplung der Federabschnitte (6) zu lösen.

11. Chirurgisches Handinstrument (1) nach einem der vorstehenden Ansprüche
**dadurch gekennzeichnet, dass**
die Winkelposition des Längsschlitzes (11) am Lagerauge (10) in Abhängigkeit der zumindest in Konstruktionslage sich ergebenden Bogenform der Blattfedern (6) derart bestimmt ist, dass bei Drücken zumindest jener Blattfeder (6) mit Scharnierstift (9) vorzugsweise an einer vordefinierten und/oder markierten Blattfederstelle in Richtung hin zur gegenüberliegenden Blattfeder (6) mit geschlitztem Lagerauge (10) das Lagerauge (10) durch dabei eintretendes sich Biegen der zugehörigen Blattfeder (6) gleichzeitig gedreht wird, bis der Scharnierstift (9) durch den Längsschlitz (11) hindurch aus dem Lagerauge (10) gleitet.

12. Chirurgisches Handinstrument (1) nach einem der vorstehenden Ansprüche
**dadurch gekennzeichnet, dass**
der Schlitz (11) auf jener Längsseite der zugehörigen Blattfeder (6) öffnet, welche der gegenüberliegenden Blattfeder (6) zugewandt ist.

13. Chirurgisches Handinstrument (1) nach einem der vorstehenden Ansprüche
**dadurch gekennzeichnet, dass**
der Scharnierstift (9) in montiertem Zustand des Scharniergelenks (8) drehbar mit dem Lagerauge (10) verbunden ist, so dass eine aus einem Zusammendrücken der Griffabschnitte (3) resultierende Rückstellkraft mit Verringerung des Winkels zwischen der beiden Griffabschnitte (3) zumindest im Wesentlichen linear zunimmt.

## Claims

1. A surgical handheld instrument (1) of the tongs or scissors type comprising the following parts:
two lever elements (2) hinged to each other, each of them including a grip portion (3) on one side of a lever element hinge (5) and a jaw portion (4) on the other side of the lever element hinge (5),
two leaf springs (6) which are proximally connected to each of the two grip portions (3) as spring portions extending each of the latter and which are coupled to each other at their proximal free ends via a hinged joint (8),
**characterized in that**
the hinged joint (8) includes a hinge pin (9) at the free end of the one leaf spring (6) and at least one bearing eye (10) at the free end of the other leaf spring (6),
wherein
the bearing eye (10) includes a slit (11) extending in the axial direction of the bearing eye for radial insertion of the hinge pin (9) into the bearing eye (10).

2. The surgical handheld instrument (1) according to claim 1,
**characterized in that**
the longitudinal slit (11) is placed at an angular position unlike the most proximal angular position of the bearing eye (10) preferably within a distal angular position area and more preferably substantially opposite to the most proximal angular position of the bearing eye (10).

3. The surgical handheld instrument (1) according to claim 1 or 2,
**characterized in that**
the longitudinal slit (11) extends diagonally within the bearing eye (10) relative to the axis of the bearing eye so that at least one spring portion (6) has to be twisted during radial insertion of the hinge pin (9) into the bearing eye (10).

4. The surgical handheld instrument (1) according to any one of the preceding claims,
**characterized in that**
the slit width is smaller than the maximum diameter of the hinge pin (9).

5. The surgical handheld instrument (1) according to claim 4,
**characterized in that**
the bearing eye (10) can be resiliently widened.

6. The surgical handheld instrument (1) according to claim 4 or 5,
**characterized in that**
the hinge pin (9) includes at least one flattened portion (9a), wherein the diameter of the hinge pin in the area of the at least one flattened portion substantially corresponds to or is smaller than the slit width.

7. The surgical handheld instrument (1) according to any one of the preceding claims,
**characterized in that**
at least one of the spring portions (6), preferably the spring portion (6) including the end-side bearing eye (10), in its longitudinal central portion bears a marking (6a) on the side thereof facing away from the other spring portion (6), wherein in the area of this marking an external pressure has to be manually applied for uncoupling the proximal hinge (8) which is immediately resulting therefrom.

8. The surgical handheld instrument (1) according to claim 5 and 6,
**characterized in that**
the angular position of the flattened portion (9a) relative to the angular position of the slit (11) is selected so that,
- when the surgical instrument is actuated, no conformity of the two angular positions is possible and,
- when pressure is applied to at least one spring portion (6), conformity of the two angular positions is achieved at the marking (6a) for uncoupling the proximal hinge (8).

9. The surgical handheld instrument (1) according to any one of the preceding claims,
**characterized in that**
the grip portions (6)
- exhibit a pitch circle cross-section smaller than a semi-circle,
- on the sides facing each other include cavities in which interacting components of a circulation barrier (3b) are at least partially accommodated and,
- when the circulation barrier (3b) is locked in place, define a gap or clearance (3a) between themselves the gap width of which forms a full circle having a substantially constant radius in interaction with the two pitch circles.

10. The surgical handheld instrument (1) according to anyone of the preceding claims,
**characterized in that**
the spring portions (6) are biased against each other in the mounted state of the hinged joint (8) so that the hinge pin (9) is retained in the bearing eye (10) by the biasing force and has to be displaced against the biasing force out of the slit (11) so as to release the hinge coupling of the spring portions (6).

11. The surgical handheld instrument (1) according to any one of the preceding claims,
**characterized in that**
the angular position of the longitudinal slit (11) at the bearing eye (10) is defined as a function of the curved shape of the leaf springs (6) resulting at least in the starting position such that, when at least the leaf spring (6) including the hinge pin (9) is forced preferably at a predefined and/or marked leaf spring position toward the opposite leaf spring (6) including the slotted bearing eye (10), the bearing eye (10) is simultaneously rotated by an occurring bending of the pertinent leaf spring (6), until the hinge pin (9) slides through the longitudinal slit (11) out of the bearing eye (10). n.

12. The surgical handheld instrument (1) according to any one of the preceding claims,
**characterized in that**
the slit (11) opens on the longitudinal side of the pertinent leaf spring (6) that is facing the opposite leaf spring (6).

13. The surgical handheld instrument (1) according to any one of the preceding claims,
**characterized in that**
in the mounted state of the hinged joint (8) the hinge pin (9) is rotatably connected to the bearing eye (10) so that a resetting force resulting from compression of the grip portions (3) at least substantially linearly increases upon reduction of the angle between the two grip portions (3).

## Revendications

1. Instrument à main chirurgical (1) de type pince ou ciseau, qui comporte les pièces suivantes :
deux éléments levier (2) reliés l'un à l'autre par charnière avec respectivement une partie de préhension (3) d'un côté d'une charnière d'élément levier (5) et une partie de mâchoire (4) de l'autre côté de la charnière d'élément levier (5),
deux ressorts à lame (6), qui se raccordent de façon proximale à chacune des deux parties de préhension (3) en tant que parties de ressort prolongeant respectivement celles-ci et sont couplés l'un à l'autre à leurs extrémités libres proximales par le biais d'une charnière (8),
**caractérisé en ce que**
la charnière (8) présente un axe de charnière (9) à l'extrémité libre de l'un ressort à lame (6) et au moins un oeil d'appui (10) à l'extrémité libre de l'autre ressort à lame (6),
dans lequel l'oeil d'appui (10) présente une encoche (11) s'étendant dans la direction de l'axe de l'oeil d'appui pour l'introduction radiale de l'axe de charnière (9) dans l'oeil d'appui (10).

2. Instrument à main chirurgical (1) selon la revendication 1,
**caractérisé en ce que**
l'encoche longitudinale (11) est placée dans une position angulaire différente de la position angulaire la plus proximale de l'oeil d'appui (10) de préférence dans une plage de position angulaire distale et de manière davantage préférée sensiblement en face de la position angulaire la plus proximale de l'oeil d'appui (10).

3. Instrument à main chirurgical (1) selon la revendication 1 ou 2,
**caractérisé en ce que**
l'encoche longitudinale (11) s'étend dans l'oeil d'appui (10) de manière oblique par rapport à l'axe de l'oeil d'appui, si bien que lors de l'introduction radiale de l'axe de charnière (9) dans l'oeil d'appui (10) au moins une partie de ressort (6) doit être torsadée.

4. Instrument à main chirurgical (1) selon l'une quelconque des revendications précédentes,
**caractérisé en ce que**
la largeur de l'encoche est inférieure au diamètre maximum de l'axe de charnière (9).

5. Instrument à main chirurgical (1) selon la revendication 4,
**caractérisé en ce que**
l'oeil d'appui (10) peut être élargi de manière élastique.

6. Instrument à main chirurgical (1) selon la revendication 4 ou 5,
**caractérisé en ce que**
l'axe de charnière (9) présente au moins un aplatissement (9a), dans lequel le diamètre de l'axe de charnière dans la zone de l'au moins un aplatissement correspond sensiblement à la largeur de l'encoche ou est inférieur.

7. Instrument à main chirurgical (1) selon l'une quelconque des revendications précédentes,
**caractérisé en ce que**
au moins une des parties de ressort (6), de préférence la partie de ressort (6) avec l'oeil d'appui (10) côté extrémité dans sa partie médiane longitudinale porte un marquage (6a) au niveau de son côté opposé à l'autre partie de ressort (6), dans la zone duquel une force de compression extérieure doit être appliquée manuellement pour un découplage en résultant immédiatement de la charnière proximale (8).

8. Instrument à main chirurgical (1) selon la revendication 5 et 6,
**caractérisé en ce que**
la position angulaire de l'aplatissement (9a) par rapport à la position angulaire de l'encoche (11) est choisie de telle sorte que
- lors d'un actionnement de l'instrument chirurgical, aucune correspondance des deux positions angulaires n'est possible et
- lors d'une application d'une force de compression de l'au moins une partie de ressort (6) sur le marquage (6a), une correspondance des deux positions angulaires est obtenue pour un découplage de la charnière proximale (8).

9. Instrument à main chirurgical (1) selon l'une quelconque des revendications précédentes,
**caractérisé en ce que**
les parties de préhension (6)
- présentent respectivement une section transversale en forme de cercle partiel inférieure à un demi-cercle,
- ont respectivement des cavités au niveau des côtés tournés l'un vers l'autre, dans lesquelles des composants coopérant d'un blocage périphérique (3b) sont reçus au moins en partie et
- définissent entre elles lorsque le blocage périphérique (3b) est enclenché, un interstice ou une distance (3a), dont la largeur d'interstice forme en coopération avec les deux cercles partiels un cercle entier avec un rayon sensiblement constant.

10. Instrument à main chirurgical (1) selon l'une quelconque des revendications précédentes
**caractérisé en ce que**
les parties de ressort (6) sont précontraintes l'une contre l'autre à l'état monté de la charnière (8), si bien que l'axe de charnière (9) est maintenu dans l'oeil d'appui (10) par la force de précontrainte et doit être déplacé contre la force de précontrainte hors de l'encoche (11) pour desserrer le couplage de charnière des parties de ressort (6).

11. Instrument à main chirurgical (1) selon l'une quelconque des revendications précédentes
**caractérisé en ce que**
la position angulaire de l'encoche longitudinale (11) au niveau de l'oeil d'appui (10) est déterminée en fonction de la forme d'arc des ressorts à lame (6) résultant au moins dans la couche de construction de telle sorte que lors de pressions de l'au moins un ressort à lame (6) avec axe de charnière (9) de préférence au niveau d'un endroit du ressort à lame prédéfini et/ou marqué en direction du ressort à lame (6) lui faisant face avec oeil d'appui (10) encoché, l'oeil d'appui (10) est tourné en même temps par la flexion des ressorts à lame (6) afférents apparaissant ce faisant, jusqu'à ce que l'axe de charnière (9) glisse par l'encoche longitudinale (11) hors de l'oeil d'appui (10).

12. Instrument à main chirurgical (1) selon l'une quelconque des revendications précédentes
**caractérisé en ce que**
l'encoche (11) s'ouvre sur le côté longitudinal du ressort à lame (6) afférent, qui est tourné vers le ressort à lame opposé (6).

13. Instrument à main chirurgical (1) selon l'une quelconque des revendications précédentes
**caractérisé en ce que**
l'axe de charnière (9) à l'état monté de la charnière (8) est relié à l'oeil d'appui (10) de manière rotative, si bien qu'une force de rappel résultant d'une compression des parties de préhension (3) augmente avec la diminution de l'angle entre les deux parties de préhension (3) au moins de manière sensiblement linéaire.
